# EUROPEAN PATENT SPECIFICATION

(11) **EP 4 389 103 B1**
(45) Date of publication and mention of the grant of the patent: **17.06.2026**
(21) Application number: 22857911.6
(22) Date of filing: 19.08.2022
(51) Int. Cl.: A61K 9/08, A61K 9/00, A61K 31/724, A61P 21/02

(54) **PACKAGED DRUG CONTAINING MERCAPTOCYCLODEXTRIN DERIVATIVE OR PHARMACEUTICALLY ACCEPTABLE SALT THEREOF AND PRESERVATION METHOD**
VERPACKTES ARZNEIMITTEL MIT MERCAPTOCYCLODEXTRINDERIVAT ODER PHARMAZEUTISCH UNBEDENKLICHEM SALZ DAVON UND KONSERVIERUNGSVERFAHREN
MÉDICAMENT EMBALLÉ CONTENANT UN DÉRIVÉ DE MERCAPTOCYCLODEXTRINE OU UN SEL PHARMACEUTIQUEMENT ACCEPTABLE CORRESPONDANT ET PROCÉDÉ DE CONSERVATION

(30) Priority: 19.08.2021 CN 202110952660
(43) Date of publication of application: 26.06.2024
(73) Proprietor: Beijing Fresenius Kabi Pharmaceutical Co., Ltd., Beijing 100028 (CN)
(72) Inventor: XU, Wenjing, Wuhan, Hubei 430000 (CN); LIU, Weitu, Wuhan, Hubei 430000 (CN); ZHANG, Yongxia, Wuhan, Hubei 430000 (CN); XIE, Ting, Wuhan, Hubei 430000 (CN); ZHANG, Yijuan, Wuhan, Hubei 430000 (CN)
(74) Representative: Fresenius Kabi Deutschland GmbH
(86) International application number: PCT/CN2022/113467
(87) International publication number: WO 2023/020602

(56) References cited:
- WO-A1-2020/028448
- WO-A1-2021/039084
- CN-A- 110 638 751
- CN-A- 112 538 123
- CN-A- 112 538 124
- CN-A- 112 933 040
- US-A1- 2021 000 739

## Description

### Technical Field

The present disclosure relates to technical field of medicine, and in particular to a packaged drug containing a mercaptocyclodextrin derivative or a pharmaceutically acceptable salt thereof and a method for preserving the same.

### Background Art

A neuromuscular blocking agent (NMBA), also called a muscle relaxant, is routinely used during the administration of anaesthetic to facilitate endotracheal intubation and to allow surgical access to body cavities, in particular the thorax and abdomen, without hindrance from voluntary or reflex muscle movement. The neuromuscular blocking agents are also used in the care of critically-ill patients undergoing intensive therapy, to facilitate compliance with mechanical ventilation when sedation and analgesia alone have proved inadequate.

The neuromuscular blocking agents may be divided into two categories: depolarizing and non-depolarizing. Depolarizing neuromuscular blocking agents bind to nicotinic acetylcholine receptors (nAChRs) at the neuromuscular junction in a way similar to that of the endogenous neurotransmitter acetylcholine. They stimulate an initial opening of the ion channel, producing contractions known as fasciculations. Since these drugs are broken down only relatively slowly by cholinesterases, compared to the very rapid hydrolysis of acetylcholine by acetylcholinesterases, they bind for a much longer period than acetylcholine, causing sustained depolarization of the end-plate and hence neuromuscular anaesthesia. Succinylcholine (suxamethonium) is a known example of a depolarizing neuromuscular blocking agent. Non-depolarizing neuromuscular blocking agents, also called competitive muscle relaxants, compete with acetylcholine for binding to muscle nicotinic acetylcholine receptors, but unlike depolarizing neuromuscular blocking agents, they do not activate the channel. They block the activation of the channel by acetylcholine and hence prevent cell membrane depolarization, and as a result, the muscle will become flaccid. Most of the clinically-used neuromuscular blocking agents belong to the non-depolarizing neuromuscular blocking agents. These include tubocurarine, atracurium, (cis)atracurium, mivacurium, rocuronium, vecuronium, etc.

When the neuromuscular blocking agents are used, residual neuromuscular blocking effect after surgery may occur, causing serious hypoxia and even upper respiratory obstruction in patients, which are important factors for causing complications and death of the patients during peri-anaesthesia. When a surgical operation is ended or a period of intensive care is ended, a reversal agent of neuromuscular blocking agents is often given to the patient to assist the recovery of muscle function thereof.

International patent application WO2001/040316 discloses use of a mercaptocyclodextrin derivative and a pharmaceutically acceptable salt thereof for the reversal of drug-induced neuromuscular block, and particularly 6-mercaptocyclodextrin derivatives of a structure of the following formula or a pharmaceutically acceptable salt thereof: 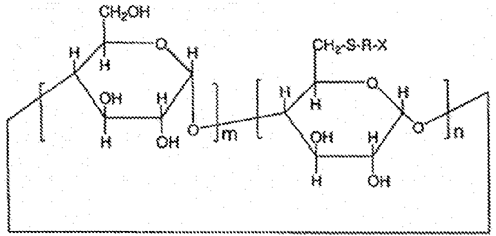 where m is 0 to 7, n is 1 to 8, and m+n=7 or 8;
R is C₁₋₆ alkylene which is unsubstituted or substituted with 1, 2 or 3 OH groups;
X is COOH, CONHR₁, NHCOR₂, SO₂OH, O(CH₂-CH₂-O)_{q}-H, OH or tetrazol-5-yl;
R₁ is H or (C₁₋₃)alkyl;
R₂ is carboxyphenyl;
q is 1, 2 or 3; and
when X is COOH, R is (CH₂)ₒ-phenylene-(CH₂)ₚ-, where o and p are each independently 0 to 4. It is worth mentioning that Sugammadex is a modified mercaptocyclodextrin derivative, and has a chemical name of 6-perdeoxy-6-per(2-carboxyethyl)thio-γ-cyclodextrin. Sugammadex sodium injection developed by MSD company was approved for marketing under the trade name of Bridion^{®} in the European Union in 2008, for reversing neuromuscular blocking effects induced by the non-depolarizing neuromuscular blocking agents rocuronium and vecuronium. In 2017, Bridion was approved for marketing in China, and current marketing specifications include 2 mL : 200 mg and 5 mL : 500 mg. Clinically, for adults, Bridion is used to antagonize rocuronium or vecuronium-induced neuromuscular block; and for children and adolescents (2~17 years), it is only recommended for conventional rocuronium-induced block. It is believed that after being injected, sugammadex sodium forms complexes with the neuromuscular blocking agent rocuronium or vecuronium, thereby reducing the number of neuromuscular blocking agents binding to the nicotinic receptors at the neuromuscular junction, and achieving the effect of rapidly reversing neuromuscular block.

US 2021/000739 A1 discloses a method to prepare pharmaceutical compositions of sugammadex, to pharmaceutical compositions of sugammadex and uniform pharmaceutical batches of said compositions. CN110638751A discloses a stable sugammadex sodium injection and a preparation method thereof.

However, the prior art still lacks intensive research on the formulation stability of such mercaptocyclodextrin derivative or a pharmaceutically acceptable salt thereof. At present, it is still necessary to further provide a relevant packaged drug of a mercaptocyclodextrin derivative or a pharmaceutically acceptable salt thereof with stable production quality and a reduced generation amount of impurities during storage and a method for preserving the same.

### Summary

In one aspect, the present invention provides a packaged drug, which contains a container, a mercaptocyclodextrin derivative as represented by formula I or a pharmaceutically acceptable salt thereof encapsulated in the container, and an inert gas sealed in the container, wherein the residual oxygen content in the headspace of the container is in a range from greater than 3% to 10%: 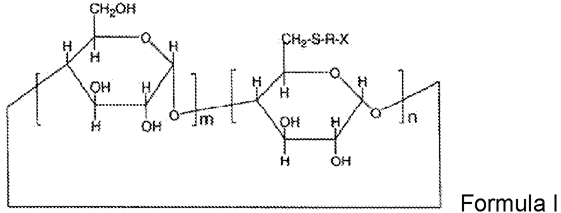 where m is an integer selected from 0 to 7, n is an integer selected from 1 to 8, and m+n=7 or 8;
R is C₁₋₆ alkylene which is unsubstituted or substituted with 1, 2 or 3 OH groups; and
X is COOH or SO₂OH.

In some embodiments, the residual oxygen content in the headspace of the container is 3.2-10%.

In some embodiments, the residual oxygen content in the headspace of the container is 3.4-5% or 5-10%.

In some embodiments, the inert gas is nitrogen.

In some embodiments, the mercaptocyclodextrin derivative or the pharmaceutically acceptable salt thereof is selected from the group consisting of:
6-perdeoxy-6-per(2-carboxyethyl)thio-γ-cyclodextrin;
6-perdeoxy-6-per(3-carboxypropyl)thio-γ-cyclodextrin;
6-perdeoxy-6-per(2-carboxypropyl)thio-γ-cyclodextrin; and
6-perdeoxy-6-per(2-sulfoethyl)thio-γ-cyclodextrin, and
their pharmaceutically acceptable salts.

In some embodiments, the pharmaceutically acceptable salt is an alkali metal salt or an alkaline earth metal salt.

In some embodiments, the mercaptocyclodextrin derivative or the pharmaceutically acceptable salt thereof is sugammadex sodium.

In some embodiments, the mercaptocyclodextrin derivative or the pharmaceutically acceptable salt thereof is in a form of injection.

In some embodiments, the container is a vial.

In some embodiments, the mercaptocyclodextrin derivative or the pharmaceutically acceptable salt thereof is sugammadex sodium, which is in a form of injection of 2 mL : 200 mg or 5 mL : 500 mg, the container is a vial, and the residual oxygen content in the headspace of the vial is 3.4-5% or 5-10%.

In another aspect, the present invention provides a method for preserving a mercaptocyclodextrin derivative as represented by formula I or a pharmaceutically acceptable salt thereof 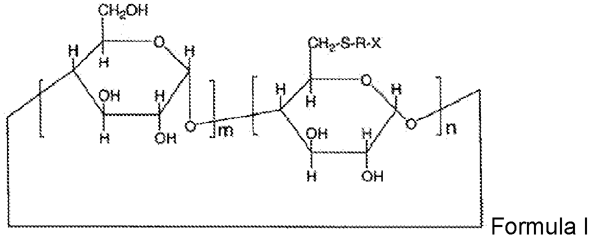
where m is an integer selected from 0 to 7, n is an integer selected from 1 to 8, and m+n=7 or 8;
   R is C₁₋₆ alkylene which is unsubstituted or substituted with 1, 2 or 3 OH groups; and
   X is COOH or SO₂OH,
the method includes the following steps:
   a) loading the mercaptocyclodextrin derivative or the pharmaceutically acceptable salt thereof into a container filled with an inert gas, and
   b) sealing the container, wherein the residual oxygen content in the headspace of the container after the sealing is in a range from greater than 3% to 10%.

In some embodiments, the residual oxygen content in the headspace of the container is 3.2-10%.

In some embodiments, the residual oxygen content in the headspace of the container is 3.4-5% or 5-10%.

In some embodiments, the inert gas is nitrogen.

In some embodiments, the mercaptocyclodextrin derivative or the pharmaceutically acceptable salt thereof is selected from the group consisting of:
6-perdeoxy-6-per(2-carboxyethyl)thio-γ-cyclodextrin;
6-perdeoxy-6-per(3-carboxypropyl)thio-γ-cyclodextrin;
6-perdeoxy-6-per(2-carboxypropyl)thio-γ-cyclodextrin; and
6-perdeoxy-6-per(2-sulfoethyl)thio-γ-cyclodextrin, and
their pharmaceutically acceptable salts.

In some embodiments, the pharmaceutically acceptable salt is an alkali metal salt or an alkaline earth metal salt.

In some embodiments, the mercaptocyclodextrin derivative or the pharmaceutically acceptable salt thereof is sugammadex sodium.

In some embodiments, the mercaptocyclodextrin derivative or the pharmaceutically acceptable salt thereof is in a form of injection.

In some embodiments, the container is a vial.

In some embodiments, the mercaptocyclodextrin derivative or the pharmaceutically acceptable salt thereof is sugammadex sodium, which is in a form of injection of 2 mL : 200 mg or 5 mL : 500 mg, the container is a vial, and the residual oxygen content in the headspace of the vial is 3.4-5% or 5-10%.

By controlling the gas and the residual oxygen content in the headspace of the formulation package of the mercaptocyclodextrin derivative or the pharmaceutically acceptable salt thereof, the present invention may provide a formulation product with a stable production quality and an improved storage quality.

### Brief Description of Drawings

The present invention may be better understood with reference to the following detailed description in combination with the drawings.
FIG. 1 shows superposed chromatograms of a sugammadex sodium injection before and after oxygen forced degradation.
FIG. 2 shows superposed chromatograms of a sugammadex sodium injection before and after thermal forced degradation.
FIG. 3 shows amounts of impurities present in different samples with relative retention time of 0.65.
FIG. 4 shows amounts of impurities present in different samples with relative retention time of 0.75.
FIG. 5 shows amounts of impurities present in different samples with relative retention time of 1.4.

### Detailed Description of Embodiments

Unless indicated to the contrary in the context, the terms in the present invention have the following meanings.

The terms "comprising", "containing" and "including" should be interpreted as open-ended, indicating the presence of elements recited but not excluding the presence, occurrence, or addition of any other element or elements which are not recited.

All ranges recited herein include the endpoints, including those that recite a range between two values, unless otherwise stated to the contrary. Whether so indicated or not, all values recited herein include the degree of expected experimental error, technique error, and instrument error for a given technique used to measure the value.

The term "cyclodextrin" is a general term for a series of cyclic oligosaccharides generated from amylose under the action of cyclodextrin glucosyltransferases, and usually contains 6 or more α-D-glucopyranose units, with various glucose units being linked into a ring by 1,4-α glycosidic bonds. Due to such cyclic arrangement, cyclodextrin molecules are slightly tapered rings, with a rim being hydrophilic and a cavity being hydrophobic. The cyclodextrin cavity can provide hydrophobic binding sites, for inclusion of various suitable guests; and the hydrophilic rim makes it water-soluble. Cyclodextrins consisting of 7 and 8 glucopyranose units are known as β- and γ-cyclodextrins, respectively.

In the present invention, the mercaptocyclodextrin derivative has a structure as represented by formula I, 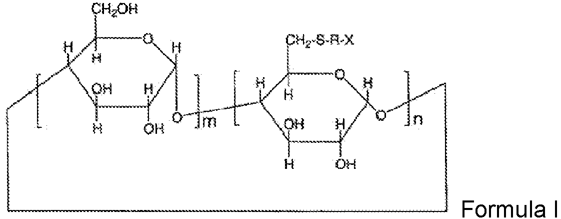 where
m is an integer selected from 0 to 7, n is an integer selected from 1 to 8, and m+n=7 or 8; particularly, m is 0 and n is 7 or 8; and more particularly, m is 0 and n is 8;
R is C₁₋₆ alkylene which is unsubstituted or substituted with 1, 2 or 3 OH groups; particularly, R is C₁₋₆ alkylene; and more particularly, R is C₁₋₃ alkylene; and
X is COOH or SO₂OH; and particularly, X is COOH.

The term "alkylene" refers to a bivalent straight or branched saturated aliphatic hydrocarbon group having a number of carbon atoms within a specified range. For example, "C₁₋₆ alkylene" refers to any one of C₁ to C₆ straight or branched alkylene. Examples may include C₁ to C₂ alkylene, C₁ to C₃ straight or branched alkylene, C₁ to C₄ straight or branched alkylene, C₁ to C₅ straight or branched alkylene; particularly C₁ to C₃ straight or branched alkylene; more particularly ethylene.

In some embodiments, in the structure of formula I, m is 0 and n is 7 or 8; R is C₁₋₃ alkylene; X is COOH or SO₂OH. Examples may include, but are not limited to, 6-perdeoxy-6-per(2-carboxyethyl)thio-γ-cyclodextrin, 6-perdeoxy-6-per(3-carboxypropyl)thio-γ-cyclodextrin, 6-perdeoxy-6-per(2-carboxypropyl)thio-γ-cyclodextrin, and 6-perdeoxy-6-per(2-sulfoethyl)thio-γ-cyclodextrin.

The term "pharmaceutically acceptable salt" refers to salts that are not biologically or otherwise undesirable (for example, neither toxic nor harmful to recipients thereof). When the compound of formula I contains one or more acidic or basic groups, the present invention further includes a pharmaceutically acceptable salt. Therefore, the compound of formula I containing the acidic group (for example, -COOH or -SO₂OH) may be used as, for example, but not limited to, an alkali metal salt, an alkaline earth metal salt or an ammonium salt, particularly an alkali metal salt. Examples may include lithium salts, sodium salts, potassium salts, calcium salts, magnesium salts, or salts with ammonia or organic amines (for example, ethylamine, ethanolamine, triethanolamine, or amino acids), particularly sodium salts and potassium salts. The pharmaceutically acceptable salts thereof may be obtained from the compound of formula I by customary methods known to those skilled in the art, for example, through combination with organic or inorganic acids or bases in a solvent or dispersant, or through anion exchange or cation exchange with other salts.

The term "sugammadex sodium" is a compound with CAS No. 343306-79-6; the chemical name is 6-perdeoxy-6-per(2-carboxyethyl)thio-γ-cyclodextrin sodium salt, the molecular formula is C₇₂H₁₀₄O₄₈S₈Na₈, and the molecular weight is 2178.01. A structural formula thereof is as follows:

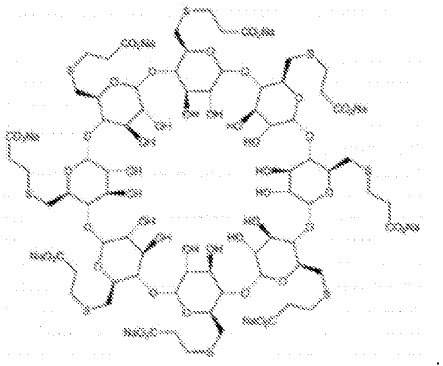

Sugammadex sodium will be oxidized and degraded in an aerobic environment. Meanwhile, as the chemical structure of sugammadex sodium is a cavity structure, sugammadex sodium is easy to chelate metal ions (e.g., iron, arsenic, nickel, manganese, etc.) from excipients, preparation production equipment, packaging materials and the like. In an aerobic environment, the presence of trace metal ions may catalyze the oxidative degradation of sugammadex sodium, causing significant increase of impurities of the sugammadex sodium injection, and significant change in the color of the liquid medicine.

In the present invention, the residual oxygen content in the headspace of the container has a significant impact on chemical properties of the encapsulated mercaptocyclodextrin derivative or a pharmaceutically acceptable salt thereof. The inventors found that reduction of the residual oxygen content in the headspace contributes to reducing generation of impurities, while an extremely low residual oxygen content in the headspace also brings about disadvantageous and extremely high requirements to a production process, can hardly be applied to the production on a production line, and causes a significant increase in production costs. In the present invention, suitable residual oxygen contents in the headspace of the container range from greater than 3% to 10%. More specifically, the residual oxygen content in the headspace of the container may be, for example, 3.2%, 3.3%, 3.4%, 3.5%, 3.8%, 4%, 4.5%, 4.8%, 5%, 5.5%, 6%, 6.5%, 7%, 7.5%, 8%, 8.5%, 9%, 9.2%, 9.5% or 10%. In some embodiments, the residual oxygen content in the headspace of the container is 3.2-10%. In some further embodiments, the residual oxygen content in the headspace of the container is 3.4-10%. In some further embodiments, the residual oxygen content in the headspace of the container is 4-10%. In some further embodiments, the residual oxygen content in the headspace of the container is 3.4-5%. In some further embodiments, the residual oxygen content in the headspace of the container is 5-10%. For the residual oxygen content in the headspace of the container, although these ranges and specific percentages above are listed, those skilled in the art should understand that the ranges within which the present invention may be implemented also include, for example, 3.2%-10%, 3.4%-10%, 4%-10%, 4.5%-10%, 4.8%-10%, or 5%-10%. In some further aspects, the residual oxygen content in the headspace of the container may be, for example, from greater than 3% to 9.5%, 3.2%-9.5%, 3.4%-9.5%, 4%-9.5%, 4.5%-9.5%, 4.8%-9.5%, or 5%-9.5%. In some further aspects, the residual oxygen content in the headspace of the container may be, for example, from greater than 3% to 9%, 3.2%-9%, 3.4%-9%, 4%-9%, 4.5%-9%, 4.8%-9%, or 5%-9%. In some further aspects, the residual oxygen content in the headspace of the container may be, for example, from greater than 3% to 8%, 3.2%-8%, 3.4%-8%, 4%-8%, 4.5%-8%, 4.8%-8%, or 5%-8%. In some further aspects, the residual oxygen content in the headspace of the container may be, for example, from greater than 3% to 7%, 3.2%-7%, 3.4%-7%, 4%-7%, 4.5%-7%, 4.8%-7%, or 5%-7%. In some further aspects, the residual oxygen content in the headspace of the container may be, for example, from greater than 3% to 6%, 3.2%-6%, 3.4%-6%, 4%-6%, 4.5%-6%, 4.8%-6%, or 5%-6%. In some further aspects, the residual oxygen content in the headspace of the container may be, for example, from greater than 3% to 5%, 3.2%-5%, 3.4%-5%, 4%-5%, 4.5%-5%, or 4.8%-5%. In some further aspects, the residual oxygen content in the headspace of the container may be, for example, from greater than 3% to 4.8%, 3.2%-4.8%, 3.4%-4.8%, 4%-4.8%, or 4.5%-4.8%.

In the present invention, the term "inert gas" refers to rare gases and inactive gases, wherein the rare gases refer to Group 18 elements on the periodic table of the elements, examples including such as helium (He), neon (Ne), argon (Ar), krypton (Kr), xenon (Xe), etc.; and the inactive gases refer to gases that do not undergo chemical reactions under certain conditions, examples including such as carbon dioxide and nitrogen. As used in the present invention, examples of the inert gases may particularly include nitrogen, carbon dioxide, and argon, and more particularly nitrogen.

In the present invention, the mercaptocyclodextrin derivative as represented by formula I or a pharmaceutically acceptable salt thereof may be present in a form suitable for storage or administration of a pharmaceutical preparation of the mercaptocyclodextrin derivative or the pharmaceutically acceptable salt thereof. Depending on different routes of administration, suitable forms may include, but are not limited to, injections, including sterile or sterilized solutions, water injections, oil injections, powder injections and so on, tablets, capsules, oral liquid dosage forms, such as solutions, syrups, emulsions, suspensions and so on, granules, ointments, suppositories, aerosols and so on. Examples are particularly in the form of injection, more particularly in the form of injection of 2 mL : 200 mg or 5 mL : 500 mg.

The term "container" may be any container suitable for preservation or administration of the mercaptocyclodextrin derivative or a pharmaceutically acceptable salt thereof. Containers which may be used in the present invention may be, for example, containers suitable for preservation or administration of the above pharmaceutical preparation forms, particularly syringes, ampoules or vials, and specifications may be, for example, 1-25 mL, such as 1 mL, 2 mL, 5 mL, 10 mL and 20 mL, particularly 2 mL and 5 mL. Other suitable containers and specifications also may be selected by those skilled in the art according to actual administration requirements, and all of such options are intended to be included within the scope of the present invention. In some embodiments, the container is a vial that is sealed with a rubber stopper and an aluminum cap.

The term "relative retention time" refers to a ratio of retention time of impurities to retention time of main components.

The term "sealing" or similar expressions may be construed as any form of sealing, as long as such sealing may isolate gases/liquids inside and outside a container. Examples include, but are not limited to, melting sealing of an ampoule, sealing of a phial or a vial with a rubber stopper and the like.

In the present invention, unless otherwise stated, % refers to the percentage of weight/weight.

### Abbreviations

RRT relative retention time
RH relative humidity

### Examples

### Preparation of Samples

Sugammadex sodium is a compound having CAS No. 343306-79-6, and is commercially available.

Reference is made to the prior art, for example, a preparation method of Bridion with the specification 2 mL : 200 mg. Preparation steps are as follows:
1) Formulation of liquid medicine
   Sugammadex sodium raw materials of a prescription volume were added to a liquid preparation tank, 80% of a total amount of preparation water was added, and they were stirred to complete dissolution. A pH value of the solution was measured. The pH was adjusted to 7.4-7.6 by adding a hydrochloric acid/sodium hydroxide solution. The solution was then made up to full volume with the preparation water.
2) Filling, stoppering and capping
   The liquid medicine was filled into clean 2-mL vials at 2 mL per vial, and the vials were stoppered and capped.
3) Sterilization
   Samples to be sterilized were loaded into a sterilization cabinet in an appropriate manner, and subjected to water bath sterilization under conditions of 121 °C and F₀≥15 minutes in a sterilization process.

### Analysis of sample impurities

Sugammadex sodium injections were prepared, and two samples were taken therefrom to analyze impurities through chromatographic analysis.

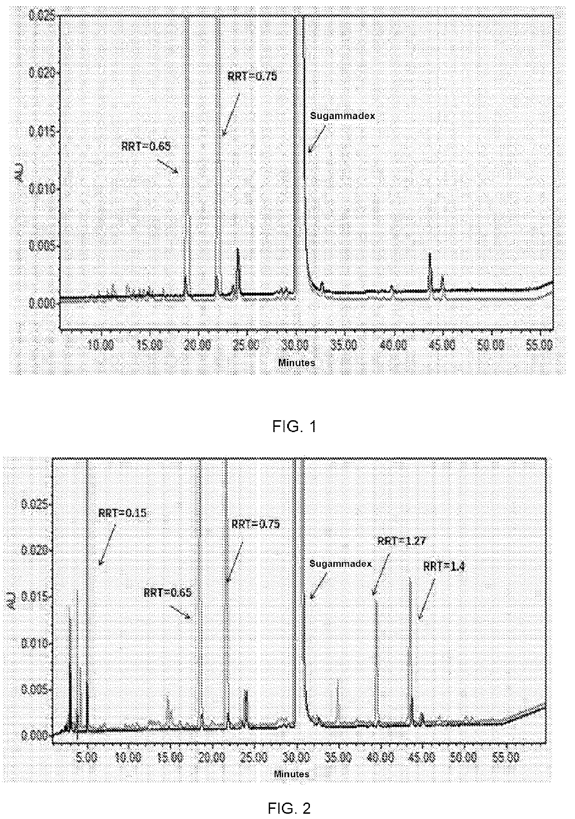

An oxygen forced degradation treatment was performed on one of the samples. The sample was subjected to a chromatographic analysis before and after the treatment, and chromatograms obtained are shown superposed in FIG. 1. A main peak and main impurity peaks are all marked in the drawing. In the above, the main peak sugammadex was observed in the sample before and after the treatment (see peak approximately at 30 minutes in FIG. 1); and two main impurities with RRTs of approximately 0.65 and 0.75 respectively were observed in the sample after the treatment.

A thermal forced degradation treatment was performed on the other sample. The sample was subjected to a chromatographic analysis before and after the treatment, and analysis chromatograms obtained are shown superposed in FIG. 2. A main peak and main impurity peaks are all marked in the drawing. In the above, the main peak sugammadex was observed in the sample before and after the treatment; and in the treated sample, in addition to two main impurities observed with RRTs of approximately 0.65 and 0.75, main impurities with RRTs of approximately 0.15, 1.27 and 1.4 respectively were also observed.

### Study of impurity contents in different preparation modes as a function of time

Three sugammadex sodium injection samples were prepared to study dissolved oxygen and residual oxygen contents. Three samples with specification 2 mL : 200 mg were prepared.

The specific preparation method of the three samples is as follows:
Sample A: Headspace was not charged with nitrogen;
   1) Formulation of liquid medicine
      Sugammadex sodium raw materials of a prescription volume were added to a liquid preparation tank, 80% of a total amount of preparation water was added, and they were stirred to complete dissolution. A pH value of the solution was measured. The pH was adjusted to 7.4-7.6 by adding a hydrochloric acid/sodium hydroxide solution. The solution was then made up to full volume with the preparation water, and was well mixed.
   2) Filling, nitrogen charging, stoppering and capping
      Filling: The liquid medicine was filled into clean 2-mL vials at 2 mL per vial, and the vials were stoppered and capped.
   3) Sterilization
      Samples to be sterilized were loaded into a sterilization cabinet in an appropriate manner, and sterilized for 15 minutes under a condition of 121 °C in a sterilization process.
Sample B: Headspace was charged with nitrogen;
   1) Formulation of liquid medicine
      Sugammadex sodium raw materials of a prescription volume were added to a liquid preparation tank, 80% of a total amount of preparation water was added, and they were stirred to complete dissolution. A pH value of the solution was measured. The pH was adjusted to 7.4-7.6 by adding a hydrochloric acid/sodium hydroxide solution. The solution was then made up to full volume with the preparation water, and was well mixed.
   2) Filling, nitrogen charging, stoppering and capping
      Filling: The solution was filled into clean 2-mL vials at 2 mL per vial; and
      Nitrogen charging: High-purity nitrogen with purity higher than 99.99% was charged to headspaces of the solution-filled vials, and the vials were stoppered and capped.
   3) Sterilization
      Samples to be sterilized were loaded into a sterilization cabinet in an appropriate manner, and subjected to water bath sterilization for 15 minutes under a condition of 121 °C in a sterilization process.
Sample C: Preparation water was bubbled with nitrogen, and the headspace was charged with nitrogen.
   1) Formulation of liquid medicine
      High-purity nitrogen with purity higher than 99.99% was blown into a beaker containing 80% of the preparation water, and the nitrogen blowing was kept for half an hour to get rid of dissolved oxygen; and
      Sugammadex sodium raw materials of a prescription volume were added to a liquid preparation tank, 80% of the preparation water having undergone nitrogen blowing for half an hour was added, and they were stirred to complete dissolution. Nitrogen was blown for protection during the stirring process. A pH value of the solution was measured. The pH was adjusted to 7.4-7.6 by adding a hydrochloric acid/sodium hydroxide solution. The solution was then made up to full volume with the preparation water, and was well mixed. Nitrogen was blown for protection during the stirring process.
   2) Filling, nitrogen charging, stoppering and capping
      Filling: The solution was filled into clean 2-mL vials at 2 mL per vial; and
      Nitrogen charging: High-purity nitrogen with purity higher than 99.99% was charged to headspaces of the solution-filled vials, and the vials were stoppered and capped.
   3) Sterilization
      Samples to be sterilized were loaded into a sterilization cabinet in an appropriate manner, and subjected to water bath sterilization for 15 minutes under a condition of 121 °C in a sterilization process.

### Stability sampling and detection

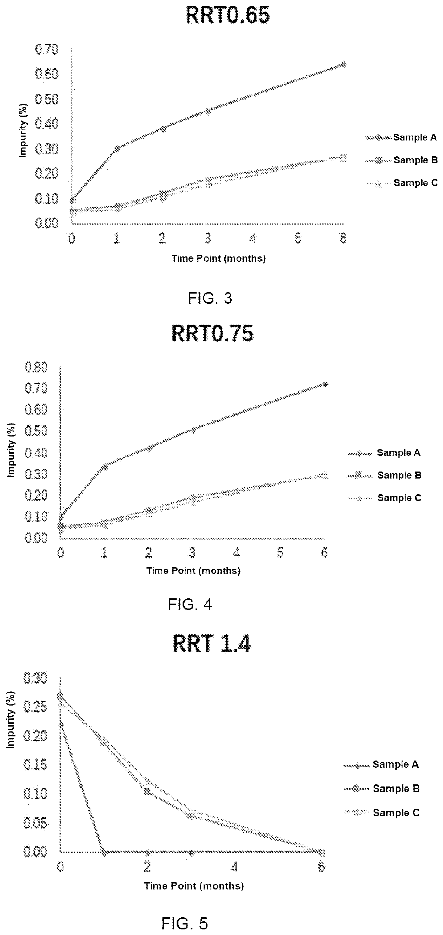

The three samples above were kept for 6 months under accelerated test conditions of 40 °C/RH 75%. Sample A, Sample B, and Sample C were continuously monitored over the 6 months, to identify amounts of impurities having relative retention time (RRT) of approximately 0.65, 0.75 and 1.4 therein, and results are shown in FIGS. 3-5. FIG. 3 and FIG. 4 show that, in Sample A not charged with nitrogen, contents of impurities with the relative retention time of approximately 0.65 and 0.75 increase faster; in Samples B and C charged with nitrogen, increase rates of impurity contents with the relative retention time of approximately 0.65 and 0.75 are significantly slowed down, and whether the water has been bubbled with nitrogen does not make an obvious difference in results. FIG. 5 shows that a lower nitrogen charging amount in the container causes faster reduction in the content of the impurity with the relative retention time of approximately 1.4.

Table 1 specifically shows the data of the amounts of impurities with the RRTs of approximately 0.65, 0.75 and 1.4 identified above by continuously monitoring the three samples, namely, Sample A, Sample B, and Sample C, over the 6 months.

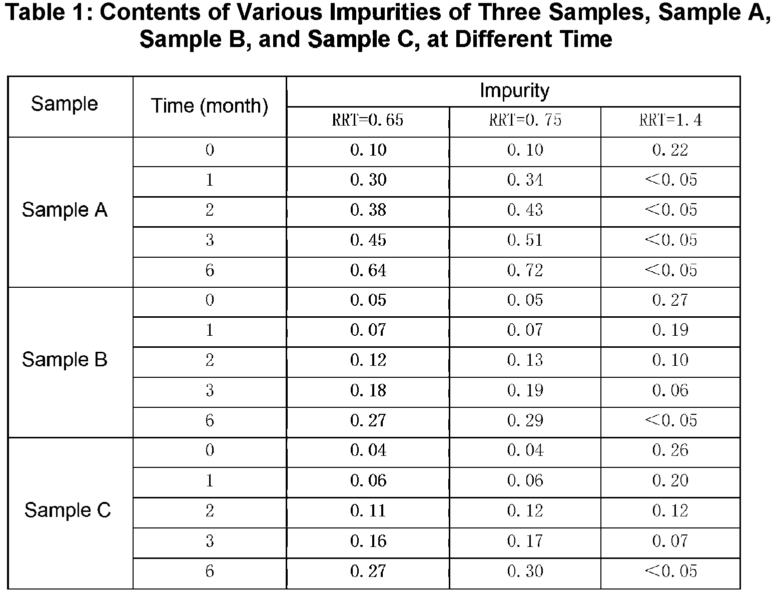

### Study of influence of oxygen in the headspace on product quality

6 batches of samples with specification 2 mL : 200 mg were formulated, and specific preparation methods are as follows.

### Sample 1

### 1) Formulation of liquid medicine

Sugammadex sodium raw materials of a prescription volume were added to a liquid preparation tank, 80% of a total amount of preparation water was added, and they were stirred to complete dissolution. A pH value of the solution was measured. The pH was adjusted to 7.4-7.6 by adding a hydrochloric acid/sodium hydroxide solution. The solution was then made up to full volume with the preparation water, and was well mixed.

### 2) Filling, nitrogen charging, stoppering and capping

Filling: The solution was filled into clean 2-mL vials at 2 mL per vial; and
nitrogen charging: High-purity nitrogen with purity higher than 99.99% was charged to headspaces of the solution-filled vials, and the vials were stoppered and capped. A residual oxygen content in the headspace of each product having been filled was 0.2%.

### 3) Sterilization

Samples to be sterilized were loaded into a sterilization cabinet in an appropriate manner, and subjected to water bath sterilization for 15 minutes under a condition of 121 °C in a sterilization process.

### Sample 2

### 1) Formulation of liquid medicine

Sugammadex sodium raw materials of a prescription volume were added to a liquid preparation tank, 80% of a total amount of preparation water was added, and they were stirred to complete dissolution. A pH value of the solution was measured. The pH was adjusted to 7.4-7.6 by adding a hydrochloric acid/sodium hydroxide solution. The solution was then made up to full volume with the preparation water, and was well mixed.

### 2) Filling, nitrogen charging, stoppering and capping

Filling: The solution was filled into clean 2-mL vials at 2 mL per vial; and
nitrogen charging: High-purity nitrogen with purity higher than 99.99% was charged to headspaces of the solution-filled vials, and the vials were stoppered and capped. A residual oxygen content in the headspace of each product having been filled was 1.5%.

### 3) Sterilization

Samples to be sterilized were loaded into a sterilization cabinet in an appropriate manner, and subjected to water bath sterilization for 15 minutes under a condition of 121 °C in a sterilization process.

### Sample 3

### 1) Formulation of liquid medicine

Sugammadex sodium raw materials of a prescription volume were added to a liquid preparation tank, 80% of a total amount of preparation water was added, and they were stirred to complete dissolution. A pH value of the solution was measured. The pH was adjusted to 7.4-7.6 by adding a hydrochloric acid/sodium hydroxide solution. The solution was then made up to full volume with the preparation water, and was well mixed.

### 2) Filling, nitrogen charging, stoppering and capping

Filling: The solution was filled into clean 2-mL vials at 2 mL per vial; and
nitrogen charging: High-purity nitrogen with purity higher than 99.99% was charged to headspaces of the solution-filled vials, and the vials were stoppered and capped. A residual oxygen content in the headspace of each product having been filled was 4.8%.

### 3) Sterilization

Samples to be sterilized were loaded into a sterilization cabinet in an appropriate manner, and subjected to water bath sterilization for 15 minutes under a condition of 121 °C in a sterilization process.

### Sample 4

### 1) Formulation of liquid medicine

Sugammadex sodium raw materials of a prescription volume were added to a liquid preparation tank, 80% of a total amount of preparation water was added, and they were stirred to complete dissolution. A pH value of the solution was measured. The pH was adjusted to 7.4-7.6 by adding a hydrochloric acid/sodium hydroxide solution. The solution was then made up to full volume with the preparation water, and was well mixed.

### 2) Filling, nitrogen charging, stoppering and capping

Filling: The solution was filled into clean 2-mL vials at 2 mL per vial; and
nitrogen charging: High-purity nitrogen with purity higher than 99.99% was charged to headspaces of the solution-filled vials, and the vials were stoppered and capped. A residual oxygen content in the headspace of each product having been filled was 9.2%.

### 3) Sterilization

Samples to be sterilized were loaded into a sterilization cabinet in an appropriate manner, and subjected to water bath sterilization for 15 minutes under a condition of 121 °C in a sterilization process.

### Sample 5

### 1) Formulation of liquid medicine

Sugammadex sodium raw materials of a prescription volume were added to a liquid preparation tank, 80% of a total amount of preparation water was added, and they were stirred to complete dissolution. A pH value of the solution was measured. The pH was adjusted to 7.4-7.6 by adding a hydrochloric acid/sodium hydroxide solution. The solution was then made up to full volume with the preparation water, and was well mixed.

### 2) Filling, stoppering and capping

Filling: The solution was filled into clean 2-mL vials at 2 mL per vial; and the vials were stoppered and capped. A residual oxygen content in the headspace of each product having been filled was 20.9%.

### 3) Sterilization

Samples to be sterilized were loaded into a sterilization cabinet in an appropriate manner, and subjected to water bath sterilization for 15 minutes under a condition of 121 °C in a sterilization process.

### Sample 6

### 1) Formulation of liquid medicine

Sugammadex sodium raw materials of a prescription volume were added to a liquid preparation tank, 80% of a total amount of preparation water was added, and they were stirred to complete dissolution. A pH value of the solution was measured. The pH was adjusted to 7.4-7.6 by adding a hydrochloric acid/sodium hydroxide solution. The solution was then made up to full volume with the preparation water, and was well mixed.

### 2) Filling, nitrogen charging, stoppering and capping

Filling: The solution was filled into clean 2-mL vials at 2 mL per vial; and
nitrogen charging: High-purity nitrogen with purity higher than 99.99% was charged to headspaces of the solution-filled vials, and the vials were stoppered and capped. A residual oxygen content in the headspace of each product having been filled was 3.4%.

### 3) Sterilization

Samples to be sterilized were loaded into a sterilization cabinet in an appropriate manner, and subjected to water bath sterilization for F₀=15 minutes under a condition of 121 °C in a sterilization process.

Samples 1-6 were placed under a high-temperature test condition (60 °C), and sampling and detection time were 0, 14 days and 1 month, respectively. And the samples were placed under accelerated test conditions (40 °C ± 2 °C / 75% RH ± 5% RH), and sampling and detection time were 1 month, 2 months, 3 months, and 6 months, respectively. Stability was observed under the two intense experimental conditions. Experimental results are shown in Table 2 and Table 3.

By comparing qualitative test results of Samples 1-6, the following may be seen:
(1) Trait: there were no obvious changes in the traits when Samples 1-6 were placed under the high-temperature test condition for 1 month and Samples 1-5 were placed under the accelerated test conditions for 6 months;
(2) Color of solution: Colors of Samples 1-6 were all darkened when Samples 1-6 were placed under the high-temperature test condition for 1 month; when Samples 1-5 were placed under the accelerated test conditions for 6 months, colors of sample 2 solutions were all slightly darkened, while the degree of color change of Sample 2 was the smallest;
(3) pH value: the pH values had no obvious changes when Samples 1-6 were placed under the high-temperature test condition for 1 month and Samples 1-5 were placed under the accelerated test conditions for 6 months; and
(4) Content and impurity:
   Chromatographic analysis conditions:
   Instrument: High performance liquid chromatograph (UV detector), chromatography column XSelect HSS C18, 3.5 µm, 4.5 x 250 mm, wavelength 210 nm, column temperature 40 °C, flow rate 0.8 mL/min, sample amount 20 µL
   Mobile phase A: 0.01% phosphoric acid solution
   Mobile Phase B: acetonitrile
   Sample solution: 4 mg/mL solution prepared with water

The contents and impurities of Samples 1-6 were detected using high performance liquid chromatography.

Table 2 is a table of changes in contents of active entities in Samples 1-6 under the high-temperature test condition and Samples 1-5 under the accelerated test conditions. As can be seen from Table 2, when Samples 1-6 were placed under the high-temperature test condition for 1 month and Samples 1-5 were placed under the accelerated test conditions for 6 months, the contents of the active entities had no obvious changes.

Table 3 is a table of changes in contents of impurities of Samples 1-6 under the high-temperature test condition and of Samples 1-5 under the accelerated test conditions. Five impurities with the RRTs of 0.60, 0.71, 1.19, 1.39, and 1.55 respectively are called as single impurity 1-5 for short in sequence hereinafter, and all the impurities are called as total impurities for short.

It can be seen from Table 3 that, when Samples 1-6 were placed under the high-temperature test condition for 1 month, a lower residual oxygen content in the headspace is followed by a slower increase rate of single impurity 1 and single impurity 2, and correspondingly a slower increase rate of the total impurities, while a slowed-down degradation rate of single impurity 5.

It can be seen from Table 3 that, in Samples 1-5, in terms of structural stability of impurities, single impurity 3 and single impurity 4 had no obvious changes when being placed under the accelerated test conditions for 6 months, and the single impurity 5 was obviously degraded when being placed under the accelerated test conditions for 6 months. In terms of amount of impurities, the amount of the single impurity 1 in Sample 1 (the residual oxygen content in the headspace was 0.2%) was gradually increased from 0.05% to 0.27% over the 6 months, the amount of the single impurity 2 was gradually increased from 0.05% to 0.29% over the 6 months, and the amount of the single impurity 5 was gradually decreased from 0.27% to <0.05% over the 6 months, then the amount of the total impurities was increased from 0.44% to 0.87%. In Sample 3 (the residual oxygen content in the headspace was 4.8%), the amount of the single impurity 1 was increased from 0.06% to 0.47% over the 6 months, the amount of the single impurity 2 was gradually increased from 0.06% to 0.52% over the 6 months, and the amount of the single impurity 5 was 0.28% at day 0, and was decreased to <0.05% in the 2^{nd} month, then the amount of the total impurities was increased from 0.58% to 1.38%. The amount of the total impurities of Samples 1-5 was gradually increased. It can thus be seen therefrom that in Sample 1, increase amplitudes of the amounts of the single impurities 1 and 2 and of the total impurities are the smallest, and the degradation rate of the single impurity 5 is also the smallest. In Sample 3, although the amounts of the single impurities 1 and 2 and of the total impurities are slightly increased, the degradation rate of the single impurity 5 is much faster than that of Sample 1, and the amounts and increase amplitudes of the single impurities 1 and 2 and of the total impurities are still lower than those of Sample 5. Therefore, the stability of Sample 3 is superior to that of Sample 5 as well as that of Sample 1. On the whole, in the samples charged with nitrogen, the lower the residual oxygen content in the headspace is, the slower the increase rates of the single impurity 1 and the single impurity 2 are, the slower the increase rate of the total impurities is, but the degradation rate of the single impurity 5 is slowed down, while the higher the residual oxygen content in the headspace is, the slower the increase rates of the single impurity 1 and the single impurity 2 are, and correspondingly, the slower the increase rate of the total impurities is, but the degradation rate of the single impurity 5 is slowed down. The slower the increase rates of the single impurity 1 and the single impurity 2 are, the faster the degradation rate of the single impurity 5 is, which is more preferable to the present invention. Therefore, in comprehensive consideration of production costs and process difficulties, the residual oxygen content in the headspace of the container is preferably in a range from greater than 3% to 10%.

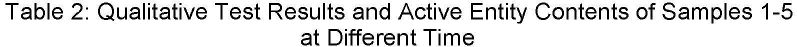

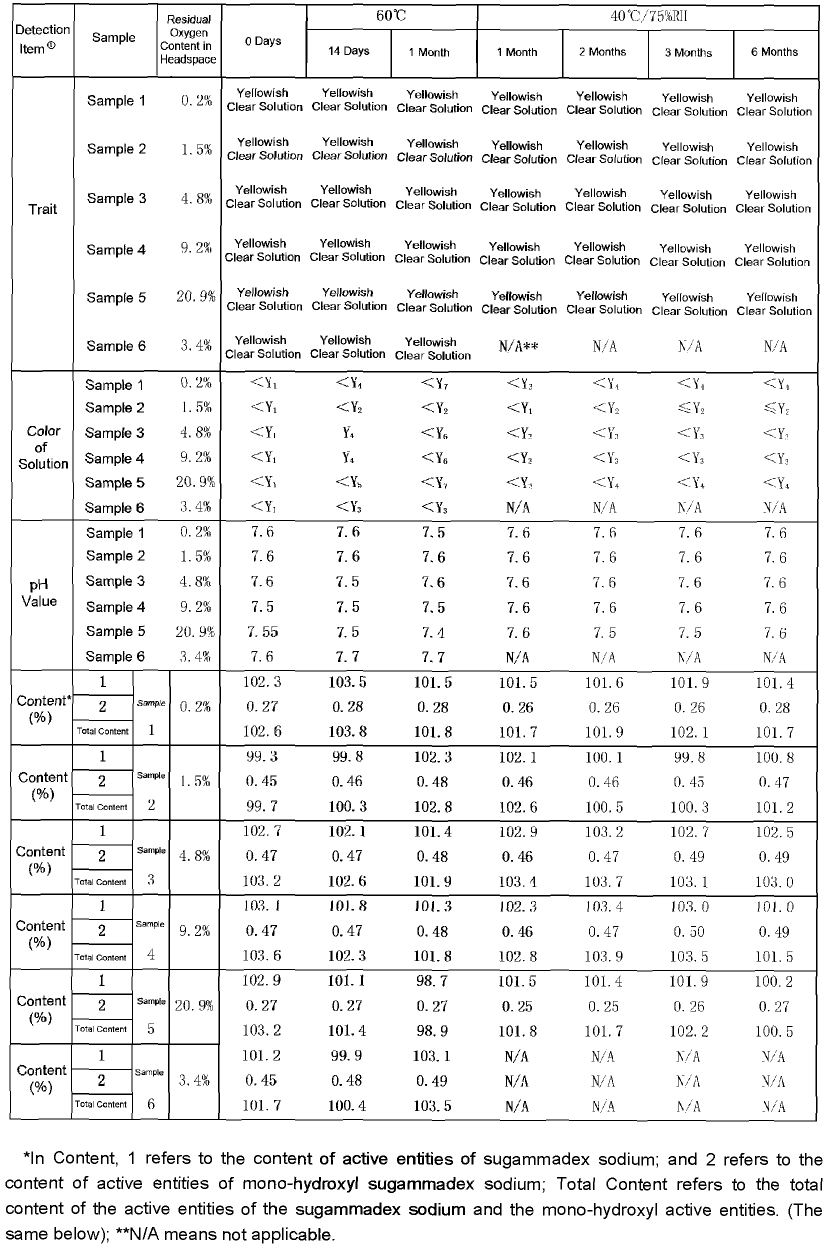

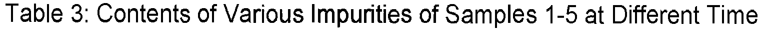

| Detection Item^{①} | | Sample 1 | Residual Oxygen Amount in Headspace | 0 Days | 60°C | | 40°C/75%RH | | | |
|---|---|---|---|---|---|---|---|---|---|---|
| | | | | | 14 Days | 1 Month | 1 Month | 2 Months | 3 Months | 6 Months |
| Single Impurity 1 (%) | RRT 0.60 | Sample 1 | 0.2% | 0. 05 | 0.15 | 0.32 | 0. 07 | 0.12 | 0.18 | 0.27 |
| | | Sample 2 | 1. 5% | 0.05 | 0.25 | 0.41 | 0. 11 | 0. 19 | 0.24 | 0. 28 |
| | | Sample 3 | 4.8% | 0.06 | 0.43 | 0.64 | 0.20 | 0.29 | 0. 35 | 0. 47 |
| | | Sample 4 | 9.2% | 0.08 | 0.60 | 0.86 | 0.26 | 0. 35 | 0.13 | 0. 56 |
| | | Sample 5 | 20. 9% | 0.10 | 0.75 | 1. 29 | 0. 30 | 0. 38 | 0.45 | 0.64 |
| | | Sample 6 | 3. 4% | 0.06 | 0.38 | 0.47 | N/A** | N/A | N/A | N/A |
| Single Impurity 2 (%) | RRT 0.71 | Sample 1 | 0.2% | 0.05 | 0.16 | 0.34 | 0. 07 | 0. 13 | 0. 19 | 0. 29 |
| | | Sample 2 | 1. 5% | 0.05 | 0.28 | 0.44 | 0. 13 | 0. 21 | 0. 25 | 0. 31 |
| | | Sample 3 | 4. 8% | 0.06 | 0.46 | 0.69 | 0. 21 | 0.31 | 0. 38 | 0.52 |
| | | Sample 4 | 9.2% | 0.08 | 0.64 | 0.93 | 0. 28 | 0. 38 | 0.48 | 0.62 |
| | | Sample 5 | 20. 9% | 0.10 | 0.82 | 1. 42 | 0. 34 | 0.43 | 0.51 | 0. 72 |
| | | Sample 6 | 3.4% | 0.06 | 0.43 | 0.51 | N/A | N/A | N/A | N/A |
| Single Impurity 3 (%) | RRT 1.19 | Sample 1 | 0.2% | <0.05 | <0.05 | 0.08 | <0. 05 | 0.06 | 0. 08 | 0.10 |
| | | Sample 2 | 1.5% | ND* | ND | 0. 06 | <0. 05 | 0.06 | 0.06 | 0.08 |
| | | Sample 3 | 4. 8% | <0. 05 | 0.05 | 0. 07 | 0. 05 | 0. 07 | 0.07 | 0. 07 |
| | | Sample 4 | 9. 2% | <0.05 | <0.05 | 0. 05 | 0. 05 | 0.06 | 0.06 | 0.06 |
| | | Sample 5 | 20.9% | <0. 05 | <0.05 | <0. 05 | <0. 05 | <0. 05 | <0.05 | <0. 05 |
| | | Sample 6 | 3.1% | ND* | 0.05 | 0.10 | N/A | N/A | N/A | N/A |
| Single Impurity 4 (%) | RRT 1.39 | Sample 1 | 0.2% | <0. 05 | 0. 07 | 11 | <0. 05 | 0. 05 | 0. 07 | 0.08 |
| | | Sample 2 | 1. 5% | <0. 05 | 0.10 | 0. 14 | 0.07 | 0.09 | 0.09 | 0.10 |
| | | Sample 3 | 4. 8% | <0. 05 | 0. 13 | 0.16 | 0. 10 | 0. 11 | 0.12 | 0. 13 |
| | | Sample 4 | 9.2% | <0.05 | 0. 14 | 0. 17 | 0.11 | 0.11 | 0.13 | 0. 13 |
| | | Sample 5 | 20. 9% | <0. 05 | 0.11 | 0. 15 | 0. 09 | 0.09 | 0.09 | 0.10 |
| | | Sample 6 | 3.4% | 0.05 | 0.10 | 0. 12 | N/A | N/A | N/A | N/A |
| Single Impurity 5 (%) | RRT 1.55 | Sample 1 | 0.2% | 0.27 | 0. 20 | 0. 12 | 0.19 | 0.10 | 0.06 | <0. 05 |
| | | Sample 2 | 1.5% | 0.22 | 0.12 | 0.10 | 0.14 | 0.07 | ND | ND |
| | | Sample 3 | 4. 8% | 0.28 | 0.06 | 0. 05 | 0.08 | ND | ND | ND |
| | | Sample 4 | 9.2% | 0.27 | <0.05 | <0. 05 | <0. 05 | ND | ND | ND |
| | | Sample 5 | 20. 9% | 0.22 | <0.05 | <0. 05 | <0. 05 | <0. 05 | <0.05 | <0. 05 |
| | | Sample 6 | 3.4% | 0.21 | ND | ND | N/A | N/A | N/A | N/A |
| Total Impurities (%) | | Sample 1 | 0.2% | | 0.75 | 1. 15 | 0. 50 | 0. 59 | 0. 71 | 0. 87 |
| | | Sample 2 | 1.5% | 0.54 | 0. 99 | 1. 53 | 0.66 | 0.88 | 0.80 | 0. 95 |
| | | Sample 3 | 4.8% | 0.56 0.58 | 1.35 | 1. 81 | 0. 80 | 0.94 | 1. 10 | 1. 38 |
| | | Sample 4 | 9.2% | 0.59 | 1. 60 | 2. 24 | 0. 87 | 1.07 | 1. 28 | 1. 45 |
| | | Sample 5 | 20. 9% | 0.60 | 1. 93 | 3. 08 | 0. 89 | 1.02 | 1. 23 | 1. 66 |
| | | Sample 6 | 3.4% | 0.55 | 1. 20 | 1. 51 | N/A | N/A | N/A | N/A |
| *ND stands for "not detected", **N/A means not applicable | | | | | | | | | | |

According to the preceding experimental results, the inventors found that the residual oxygen content in the headspace of the packaging container of the drug has a significant impact on the chemical property of the encapsulated mercaptocyclodextrin derivative or a pharmaceutically acceptable salt thereof, more particularly, the sugammadex sodium. Upon analysis, the residual oxygen content in the headspace of the Bridion product is 18%, and it is believed that it is not charged with nitrogen. The reduction of the residual oxygen content in the headspace is found to help reduce the generation of impurities. However, an extremely low residual oxygen content in the headspace (less than 3%) may also disadvantageously bring about an extremely high requirement to the production process, for example, if the ampoule is used as the container, oxygen is required for combustion during a melting sealing process, and nitrogen also will overflow, thereby causing increased difficulty in maintaining the extremely low residual oxygen content, and therefore, the extremely low residual oxygen content in the headspace (less than 3%) can hardly be applied to production on a production line, and cause significant increase in production costs. The packaging material in the present invention, such as the vials, is more suitable for the sugammadex sodium injection which needs control over the residual oxygen content in the headspace. The inventors have unexpectedly found that by controlling the residual oxygen content in the headspace of the container to be within a range from greater than 3% to 10%, particularly 3.2-10%, more particularly 3.4-5% or 5-10%, it is possible to provide a satisfactory sugammadex sodium formulation product with a stable production quality and a reduced generation amount of impurities during storage while effectively controlling the production costs. The nitrogen-charging process is stable and controllable, and thus the shelf life of the products can be prolonged.

## Claims

1. A packaged drug, which contains a container, a mercaptocyclodextrin derivative as represented by formula I or a pharmaceutically acceptable salt thereof encapsulated in the container, and an inert gas sealed in the container, wherein the residual oxygen content in the headspace of the container is in a range from greater than 3% to 10%: 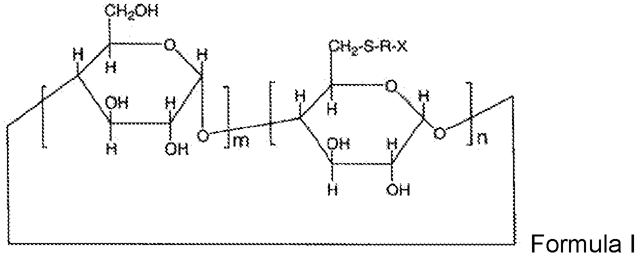
where m is an integer selected from 0 to 7, n is an integer selected from 1 to 8, and m+n=7 or 8;
R is C₁₋₆ alkylene which is unsubstituted or substituted with 1, 2 or 3 OH groups; and
X is COOH or SO₂OH.

2. The packaged drug according to claim 1, wherein the residual oxygen content in the headspace of the container is 3.2-10%, or wherein the residual oxygen content in the headspace of the container is 3.4-5% or 5-10%.

3. The packaged drug according to any one of claims 1-2, wherein the mercaptocyclodextrin derivative or the pharmaceutically acceptable salt thereof is selected from the group consisting of:
6-perdeoxy-6-per(2-carboxyethyl)thio-γ-cyclodextrin;
6-perdeoxy-6-per(3-carboxypropyl)thio-γ-cyclodextrin;
6-perdeoxy-6-per(2-carboxypropyl)thio-γ-cyclodextrin; and
6-perdeoxy-6-per(2-sulfoethyl)thio-γ-cyclodextrin, and
their pharmaceutically acceptable salts.

4. The packaged drug according to any one of claims 1-3, wherein the mercaptocyclodextrin derivative or the pharmaceutically acceptable salt thereof is sugammadex sodium.

5. The packaged drug according to any one of claims 1-4, wherein the mercaptocyclodextrin derivative or the pharmaceutically acceptable salt thereof is in a form of injection.

6. The packaged drug according to any one of claims 1-5, wherein the mercaptocyclodextrin derivative or the pharmaceutically acceptable salt thereof is sugammadex sodium, which is in a form of injection of 2 mL : 200 mg or 5 mL : 500 mg, the container is a vial, and the residual oxygen content in the headspace of the vial is 3.4-5% or 5-10%.

7. A method for preserving a mercaptocyclodextrin derivative as represented by formula I or a pharmaceutically acceptable salt thereof, 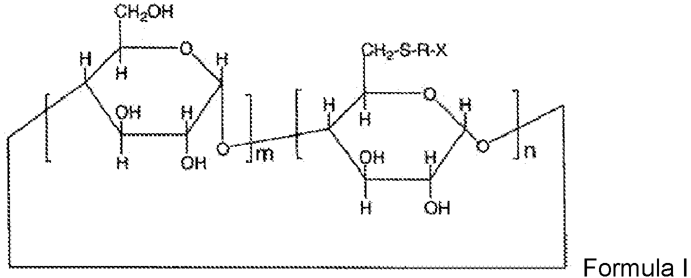
where m is an integer selected from 0 to 7, n is an integer selected from 1 to 8, and m+n=7 or 8;
R is C₁₋₆ alkylene which is unsubstituted or substituted with 1, 2 or 3 OH groups; and
X is COOH or SO₂OH,
the method comprises the following steps:
a) loading the mercaptocyclodextrin derivative or the pharmaceutically acceptable salt thereof into a container filled with an inert gas, and
b) sealing the container, wherein the residual oxygen content in the headspace of the container after the sealing is in a range from greater than 3% to 10%.

8. The method according to claim 7, wherein the residual oxygen content in the headspace of the container is 3.2-10%, or wherein the residual oxygen content in the headspace of the container is 3.4-5% or 5-10%.

9. The packaged drug according to any one of claims 1-2 or the method according to any one of claims 7-8, wherein the inert gas is nitrogen.

10. The method according to any one of claims 7-9, wherein the mercaptocyclodextrin derivative or the pharmaceutically acceptable salt thereof is selected from the group consisting of:
6-perdeoxy-6-per(2-carboxyethyl)thio-γ-cyclodextrin;
6-perdeoxy-6-per(3-carboxypropyl)thio-γ-cyclodextrin;
6-perdeoxy-6-per(2-carboxypropyl)thio-γ-cyclodextrin; and
6-perdeoxy-6-per(2-sulfoethyl)thio-γ-cyclodextrin, and
their pharmaceutically acceptable salts.

11. The packaged drug according according to any one of claims 1-3 or the method according to any one of claims 7-10, wherein the pharmaceutically acceptable salt is an alkali metal salt or an alkaline earth metal salt.

12. The method according to any one of claims 7-11, wherein the mercaptocyclodextrin derivative or the pharmaceutically acceptable salt thereof is sugammadex sodium.

13. The method according to any one of claims 7-12, wherein the mercaptocyclodextrin derivative or the pharmaceutically acceptable salt thereof is in a form of injection.

14. The packaged drug according to any one of claims 1-5 or the method according to any one of claims 7-13, wherein the container is a vial.

15. The method according to any one of claims 7-14, wherein the mercaptocyclodextrin derivative or the pharmaceutically acceptable salt thereof is sugammadex sodium, which is in a form of injection of 2 mL : 200 mg or 5 mL : 500 mg, the container is a vial, and the residual oxygen content in the headspace of the vial is 3.4-5% or 5-10%.

## Patentansprüche

1. Verpacktes Arzneimittel, das einen Behälter, ein Mercaptocyclodextrinderivat, wie durch Formel I dargestellt, oder ein pharmazeutisch unbedenkliches Salz davon, eingekapselt in dem Behälter, und ein in dem Behälter eingesiegeltes Inertgas enthält, wobei der Restsauerstoffgehalt in dem Kopfraum des Behälters in einem Bereich von mehr als 3 % bis 10 % liegt: 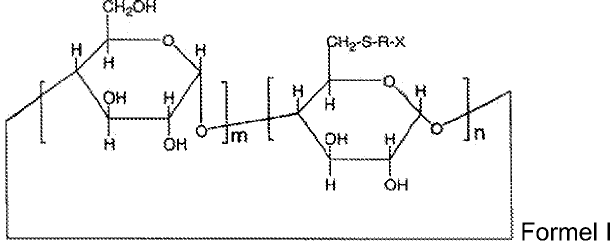
wobei m eine ganze Zahl ist, ausgewählt aus 0 bis 7, n eine ganze Zahl ist, ausgewählt aus 1 bis 8, und m+n=7 oder 8;
R C₁₋₆-Alkylen ist, das unsubstituiert oder mit 1, 2 oder 3 OH-Gruppen substituiert ist; und
X COOH oder SO₂OH ist.

2. Verpacktes Arzneimittel nach Anspruch 1, wobei der Restsauerstoffgehalt im Kopfraum des Behälters 3,2-10 % beträgt, oder wobei der Restsauerstoffgehalt im Kopfraum des Behälters 3,4-5 % oder 5-10 % beträgt.

3. Verpacktes Arzneimittel nach einem der Ansprüche 1-2, wobei das Mercaptocyclodextrinderivat oder das pharmazeutisch unbedenkliche Salz davon ausgewählt ist aus der Gruppe bestehend aus:
6-Perdeoxy-6-per(2-carboxyethyl)thio-γ-cyclodextrin;
6-Perdeoxy-6-per(3-carboxypropyl)thio-γ-cyclodextrin;
6-Perdeoxy-6-per(2-carboxypropyl)thio-γ-cyclodextrin; und
6-Perdeoxy-6-per(2-sulfoethyl)thio-γ-cyclodextrin und
ihre pharmazeutisch unbedenklichen Salze.

4. Verpacktes Arzneimittel nach einem der Ansprüche 1-3, wobei das Mercaptocyclodextrinderivat oder das pharmazeutisch unbedenkliche Salz davon Sugammadex-Natrium ist.

5. Verpacktes Arzneimittel nach einem der Ansprüche 1-4, wobei das Mercaptocyclodextrinderivat oder das pharmazeutisch unbedenkliche Salz davon in einer Injektionsform vorliegt.

6. Verpacktes Arzneimittel nach einem der Ansprüche 1-5, wobei das Mercaptocyclodextrinderivat oder das pharmazeutisch unbedenkliche Salz davon Sugammadex-Natrium ist, das in einer Injektionsform von 2 ml : 200 mg oder 5 ml : 500 mg vorliegt, der Behälter ein Fläschchen ist und der Restsauerstoffgehalt im Kopfraum des Fläschchens 3,4-5 % oder 5-10 % beträgt.

7. Verfahren zur Konservierung eines Mercaptocyclodextrinderivats, wie durch Formel I dargestellt, oder eines pharmazeutisch unbedenklichen Salzes davon, 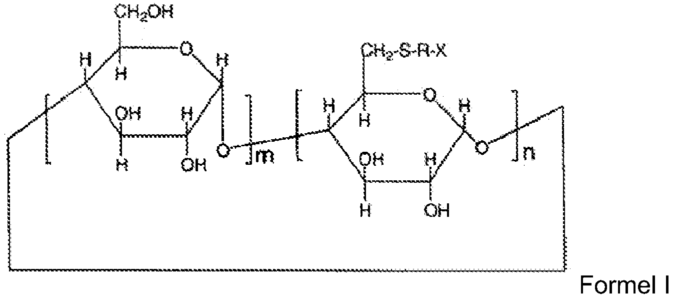
wobei m eine ganze Zahl ist, ausgewählt aus 0 bis 7, n eine ganze Zahl ist, ausgewählt aus 1 bis 8, und m+n=7 oder 8;
R C₁₋₆-Alkylen ist, das unsubstituiert oder mit 1, 2 oder 3 OH-Gruppen substituiert ist; und
X COOH oder SO₂OH ist,
wobei das Verfahren die folgenden Schritte umfasst:
a) Laden des Mercaptocyclodextrinderivats oder des pharmazeutisch unbedenklichen Salzes davon in einen Behälter, der mit einem Inertgas gefüllt ist, und
b) Versiegeln des Behälters, wobei der Restsauerstoffgehalt im Kopfraum des Behälters nach dem Versiegeln in einem Bereich von mehr als 3 % bis 10 % liegt.

8. Verfahren nach Anspruch 7, wobei der Restsauerstoffgehalt im Kopfraum des Behälters 3,2-10 % beträgt, oder wobei der Restsauerstoffgehalt im Kopfraum des Behälters 3,4-5 % oder 5-10 % beträgt.

9. Verpacktes Arzneimittel nach einem der Ansprüche 1-2 oder Verfahren nach einem der Ansprüche 7-8, wobei das Inertgas Stickstoff ist.

10. Verfahren nach einem der Ansprüche 7-9, wobei das Mercaptocyclodextrinderivat oder das pharmazeutisch unbedenkliche Salz davon ausgewählt ist aus der Gruppe bestehend aus:
6-Perdeoxy-6-per(2-carboxyethyl)thio-γ-cyclodextrin;
6-Perdeoxy-6-per(3-carboxypropyl)thio-γ-cyclodextrin;
6-Perdeoxy-6-per(2-carboxypropyl)thio-γ-cyclodextrin; und
6-Perdeoxy-6-per(2-sulfoethyl)thio-γ-cyclodextrin und
ihre pharmazeutisch unbedenklichen Salze.

11. Verpacktes Arzneimittel nach einem der Ansprüche 1-3 oder Verfahren nach einem der Ansprüche 7-10, wobei das pharmazeutisch unbedenkliche Salz ein Alkalimetallsalz oder ein Erdalkalimetallsalz ist.

12. Verfahren nach einem der Ansprüche 7-11, wobei das Mercaptocyclodextrinderivat oder das pharmazeutisch unbedenkliche Salz davon Sugammadex-Natrium ist.

13. Verfahren nach einem der Ansprüche 7-12, wobei das Mercaptocyclodextrinderivat oder das pharmazeutisch unbedenkliche Salz davon in einer Injektionsform vorliegt.

14. Verpacktes Arzneimittel nach einem der Ansprüche 1-5 oder Verfahren nach einem der Ansprüche 7-13, wobei der Behälter ein Fläschchen ist.

15. Verfahren nach einem der Ansprüche 7-14, wobei das Mercaptocyclodextrinderivat oder das pharmazeutisch unbedenkliche Salz davon Sugammadex-Natrium ist, das in einer Injektionsform von 2 ml : 200 mg oder 5 ml : 500 mg vorliegt, der Behälter ein Fläschchen ist und der Restsauerstoffgehalt im Kopfraum des Fläschchens 3,4-5 % oder 5-10 % beträgt.

## Revendications

1. Médicament conditionné, qui contient un récipient, un dérivé de mercaptocyclodextrine tel que représenté par la formule I ou un sel pharmaceutiquement acceptable de celui-ci encapsulé dans le récipient, et un gaz inerte scellé dans le récipient, dans lequel la teneur en oxygène résiduel dans l'espace de tête du récipient est dans la plage allant de plus de 3 % à 10 % : 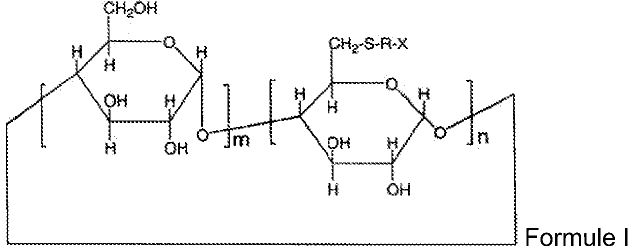
où m est un nombre entier choisi entre 0 et 7, n est un nombre entier choisi entre 1 et 8, et m+n=7 ou 8 ;
R est un alkylène en C₁₋₆ non substitué ou substitué par 1, 2 ou 3 groupes OH ; et
X est COOH ou SO₂OH.

2. Médicament conditionné selon la revendication 1, dans lequel la teneur en oxygène résiduel dans l'espace de tête du récipient est de 3,2 à 10 %, ou dans lequel la teneur en oxygène résiduel dans l'espace de tête du récipient est de 3,4 à 5 % ou de 5 à 10 %.

3. Médicament conditionné selon l'une quelconque des revendications 1 à 2, dans lequel le dérivé de mercaptocyclodextrine ou le sel pharmaceutiquement acceptable de celui-ci est choisi dans le groupe constitué de :
6-perdeoxy-6-per(2-carboxyéthyl)thio-γ-cyclodextrine ;
6-perdeoxy-6-per(3-carboxypropyl)thio-γ-cyclodextrine ;
6-perdeoxy-6-per(2-carboxypropyl)thio-γ-cyclodextrine ; et
6-perdésoxy-6-per(2-sulfoéthyl)thio-γ-cyclodextrine, et
leurs sels pharmaceutiquement acceptables.

4. Médicament conditionné selon l'une quelconque des revendications 1 à 3, dans lequel le dérivé de mercaptocyclodextrine ou le sel pharmaceutiquement acceptable de celui-ci est le sugammadex sodique.

5. Médicament conditionné selon l'une quelconque des revendications 1 à 4, dans lequel le dérivé de mercaptocyclodextrine ou le sel pharmaceutiquement acceptable de celui-ci se présente sous une forme d'injection.

6. Médicament conditionné selon l'une quelconque des revendications 1 à 5, dans lequel le dérivé de mercaptocyclodextrine ou le sel pharmaceutiquement acceptable de celui-ci est le sugammadex sodique, qui se présente sous une forme d'injection de 2 mL : 200 mg ou 5 mL : 500 mg, le récipient est un flacon, et la teneur en oxygène résiduel dans l'espace de tête du flacon est de 3,4 à 5 % ou de 5 à 10 %.

7. Procédé de conservation d'un dérivé de mercaptocyclodextrine tel que représenté par la formule I ou un sel pharmaceutiquement acceptable de celui-ci, 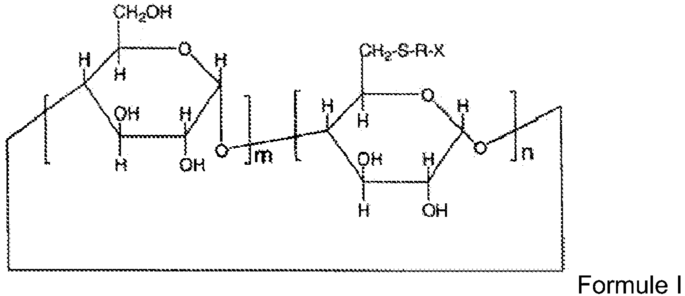
où m est un nombre entier choisi entre 0 et 7, n est un nombre entier choisi entre 1 et 8, et m+n=7 ou 8 ;
R est un alkylène en C₁₋₆ non substitué ou substitué par 1, 2 ou 3 groupes OH ; et
X est COOH ou SO₂OH,
le procédé comprend les étapes suivantes :
a) charger le dérivé de mercaptocyclodextrine ou le sel pharmaceutiquement acceptable de celui-ci dans un récipient rempli d'un gaz inerte, et
b) sceller le récipient, dans lequel la teneur en oxygène résiduel dans l'espace de tête du récipient après le scellement est dans la plage allant de plus de 3 % à 10 %.

8. Procédé selon la revendication 7, dans lequel la teneur en oxygène résiduel dans l'espace de tête du récipient est de 3,2 à 10 %, ou dans lequel la teneur en oxygène résiduel dans l'espace de tête du récipient est de 3,4 à 5 % ou de 5 à 10 %.

9. Médicament conditionné selon l'une quelconque des revendications 1 à 2 ou procédé selon l'une quelconque des revendications 7 à 8, dans lequel le gaz inerte est de l'azote.

10. Procédé selon l'une quelconque des revendications 7 à 9, dans lequel le dérivé de mercaptocyclodextrine ou le sel pharmaceutiquement acceptable de celui-ci est choisi dans le groupe constitué de :
6-perdeoxy-6-per(2-carboxyéthyl)thio-γ-cyclodextrine ;
6-perdeoxy-6-per(3-carboxypropyl)thio-γ-cyclodextrine ;
6-perdeoxy-6-per(2-carboxypropyl)thio-γ-cyclodextrine ; et
6-perdésoxy-6-per(2-sulfoéthyl)thio-γ-cyclodextrine, et
leurs sels pharmaceutiquement acceptables.

11. Médicament conditionné selon l'une quelconque des revendications 1 à 3 ou procédé selon l'une quelconque des revendications 7 à 10, dans lequel le sel pharmaceutiquement acceptable est un sel de métal alcalin ou un sel de métal alcalino-terreux.

12. Procédé selon l'une quelconque des revendications 7 à 11, dans lequel le dérivé de mercaptocyclodextrine ou le sel pharmaceutiquement acceptable de celui-ci est le sugammadex sodique.

13. Procédé selon l'une quelconque des revendications 7 à 12, dans lequel le dérivé de mercaptocyclodextrine ou le sel pharmaceutiquement acceptable de celui-ci se présente sous une forme d'injection.

14. Médicament conditionné selon l'une quelconque des revendications 1 à 5 ou procédé selon l'une quelconque des revendications 7 à 13, dans lequel le récipient est un flacon.

15. Procédé selon l'une quelconque des revendications 7 à 14, dans lequel le dérivé de mercaptocyclodextrine ou le sel pharmaceutiquement acceptable de celui-ci est le sugammadex sodique, qui se présente sous une forme d'injection de 2 mL : 200 mg ou 5 mL : 500 mg, le récipient est un flacon, et la teneur en oxygène résiduel dans l'espace de tête du flacon est de 3,4 à 5 % ou de 5 à 10 %.
